# EUROPEAN PATENT APPLICATION

(11) **EP 4 349 345 A1**
(43) Date of publication of application: **10.04.2024**
(21) Application number: 23201157.7
(22) Date of filing: 02.10.2023
(51) Int. Cl.: A61K 33/26, A61K 9/127, A61K 31/375, A61P 13/12

(54) **LIPOSOMAL COMPOSITION INCLUDING IRON SULFATE AND ITS USE**

(30) Priority: 03.10.2022 IT 202200020262
(71) Applicant: Bio-Therapic Italia S.r.l., 00193 Roma (IT)
(72) Inventor: INFORTUNA, Paolo, I-00193 Roma (IT); POLLICHIENI, Pietro, I-00193 Roma (IT)
(74) Representative: Long, Giorgio

(57) **Abstract**

The present invention relates to a liposomal composition containing an iron(II) salt, in particular iron sulfate, and the use thereof in the treatment of iron deficiency in patients suffering from chronic renal failure (also known as CKD), in particular in kidney transplant patients.

## Description

### Field of the invention

The present invention relates to a liposomal composition containing an iron(II) salt, in particular iron sulfate, and the use thereof in the treatment of iron deficiency in patients suffering from chronic renal failure (also known as CKD), in particular in kidney transplant patients.

### Background of the invention

As is known, iron plays an essential role in energy metabolism and in the synthesis of life-essential molecules, in particular the "heme" group forming part of the structure of hemoglobin, responsible for the transport of oxygen in the blood. However, the low availability of iron when taken in the form of salt or with food is also known, as well as the high toxicity thereof linked to the prooxidant effect that it can manifest if taken in excess.

Iron deficiency can be linked to a certain disease (iron-deficiency anemia), or simply to a temporary or transitory condition (e.g., pregnant women), whereby the population needing to supplement their iron deficiency with external intake is quite large.

As reported in the publication "Deficit marziale nella Malattia Renale Cronica non dialitica: dalla diagnosi al trattamento", M. E. Liberti et al., Giornale Italiano di Nefrologia, 2017, ch. 5, pp. 1-12, the management of anemia secondary to chronic renal failure (also known as CKD) is an aspect which requires special attention by nephrology specialists since it is independently associated with an increased cardiovascular and renal progression risk and a worsening of the quality of life. Taking into account that the transport of iron between the different compartments (muscle, intestines, macrophages) is ensured by transferrin and that the total amount of iron bound to transferrin is only 3 mg, in order to ensure normal erythropoiesis under physiological conditions, a turnover of iron bound to transferrin is necessary 6-7 times a day (in fact, for the production of new red blood cells about 20-25 mg of elemental iron must be conveyed to the bone marrow). In the presence of increased cytokine production, as may occur in the course of CKD, the binding capacity of transferrin is reduced and, to maintain the production of red blood cells unchanged, the daily turnover should increase up to 12 times.

Post-kidney transplant anemia (PTA) has a high prevalence (20-30%) and is associated with a worse transplant outcome, an increased cardiovascular risk, and a worsening of the quality of life. Iron deficiency (ID) in kidney transplant patients (hereinafter also referred to as KTx) is one of the main causes of PTA. Anemia has been defined as a hemoglobin (Hb) level less than 12g/dL in women and less than 13g/dL in men, in accordance with World Health Organization (WHO) criteria. Severe anemia has been defined as a hemoglobin level less than 11g/dL. PTA is associated with transplant loss and mortality, especially during the first three years.

As reported in the aforementioned work to M.E. Liberti et al., the therapeutic options for the treatment of anemia in CKD comprise iron-based preparations, ESAs, and blood transfusions. The use of oral iron-based formulations is generally considered first-line for treating iron deficiency, as they are cheaper than those for intravenous use and with quicker and more practical access by the patient. Typical indications include 100-200 mg of elemental iron per day, away from meals. However, in subjects treated with oral iron-based preparations, a prevalence of mainly gastrointestinal adverse effects (nausea, constipation or diarrhea) of about 12% has been found. Additional problems inherent to oral iron administration are the failure to correct the deficiency (poor treatment efficacy) and a generalized inflammatory state which suggests a failure to respond due to high levels of circulating hepcidin. In order to overcome such problems, intravenous administration is therefore suggested. This type of administration must necessarily occur in hospitals, resulting in discomfort for the patient.

As part of an intravenous therapy, intravenous treatment with iron gluconate has resulted in a correction of iron deficiency anemia similar to that obtained with oral iron sulfate and in a similar reduction in renal function over the 24 months of the study. However, the study was terminated prematurely due to a significant increase in serious adverse events of a cardiovascular and infectious nature. Conversely, it has been shown that the use of ferric carboxymaltose (FCM) for 24 weeks in patients with congestive heart failure (NYHA II or III), restoring the blood iron deposits, leads to an improvement in symptomatology and NYHA functional class (primary endpoints) as well as cognitive and physical *performance* (6-min walk test) and quality of life (secondary endpoints), in the absence of significant side effects. However, such intravenous treatment still has the problem of needing to be administered in hospitals and creating considerable discomfort for the patient.

Therefore, it is the object of the present invention to provide an anemia treatment in patients suffering from chronic renal failure (CKD), including post-transplant patients, which is effective, substantially free of side effects, and easy to administer even by the same patient.

### Summary of the invention

Therefore, the present invention relates to a liposomal composition comprising liposomes containing iron(II) sulfate and vitamin C, for use in the treatment of chronic renal failure.

The invention also relates to a liposomal composition comprising liposomes containing iron(II) sulfate and vitamin C, for use in the treatment of chronic renal failure in patients who have undergone a kidney transplant.

The invention further relates to a liposomal composition comprising maltodextrins and liposomes containing iron(II) sulfate and vitamin C.

### Brief description of the drawings

Figure 1 depicts a graph of: A) serum iron level, B) ferritin level, C) transferrin saturation percentage, as a function of the time of treatment with the composition of the invention;
Figure 2 depicts a graph of: A) hemoglobin (Hb) level, B) percentage of patients with ESAs, as a function of the time of treatment with the composition of the invention.

### Detailed description of the invention

In accordance with a first object, the invention relates to a liposomal composition comprising liposomes containing iron(II) sulfate and vitamin C, for use in the treatment of chronic renal failure.

In accordance with a further object of the invention, the invention relates to a liposomal composition comprising liposomes containing iron(II) sulfate and vitamin C, for use in the treatment of chronic renal failure in patients who have undergone a kidney transplant.

The liposomes used for the purposes of the present invention are, for example, those described in Italian Patent No. IT 1269946 granted on 04/16/1997 (Application No. 101994900375993 of 06/24/1995).

Liposomes according to the invention form a liposomal component consisting of phospholipid vesicles, e.g., lecithin phospholipids, containing per dose unit:
- iron(II) sulfate 20-40 mg;
- vitamin C 20-30 mg.

The composition according to the invention, in addition to the aforesaid liposomal component, comprises 300-400 mg maltodextrins per dose unit.

In a preferred embodiment, the composition according to the invention comprises, per dose unit:
- liposomes containing 30 mg iron(II) sulfate (150 mg 200 solution) and 24 mg vitamin C;
- 335 mg maltodextrins.

The composition can be in the form of granules in gastro-resistant capsules and can further contain other non-active excipients such as magnesium stearate and silicon dioxide.

The invention further relates to pharmaceutical products, dietary products, food supplements, and foods for special medical purposes (FSMPs) comprising the composition of the invention.

The term "foods for special medical purposes" means products authorized according to regulation (EU) 2016/128. Such a term refers to a product to be administered under medical supervision, thus assimilating such an FSMP to a drug.

### EXPERIMENTAL SECTION

A clinical trial was conducted in patients with CKD following kidney transplant (KTx patients).

The study evaluated efficacy, tolerability, and safety in KTx patients with blood iron deficiency anemia, treated with oral supplementation of divalent iron sulfate (DIS) associated with Vitamin C in liposomal form (composition according to the invention).

Methods: 20 KTx patients (9 males with Hb <13 g/dl and 11 females with Hb <12 g/dl, age >18 years, treatment time >6 months) with stable renal function suffering from anemia associated with iron deficiency (TSAT<20% and/or ferritin <100ng/ml) underwent oral supplementation of DIS 30 mg + Vitamin C 24 mg liposomal BID for 6 months. During the follow-up with time-points at 1, 3 and 6 months, the following were evaluated: clinical variations regarding blood iron level, dose of erythropoietin (ERI), and correction of anemia.

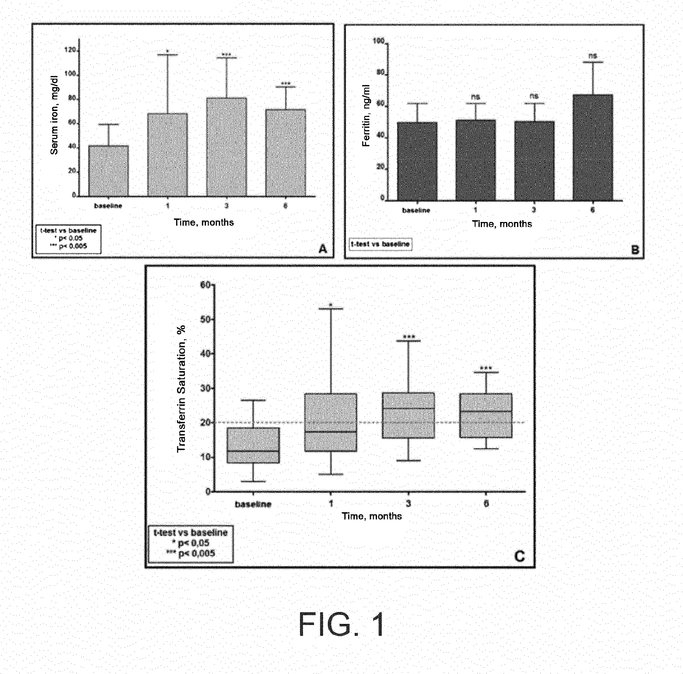

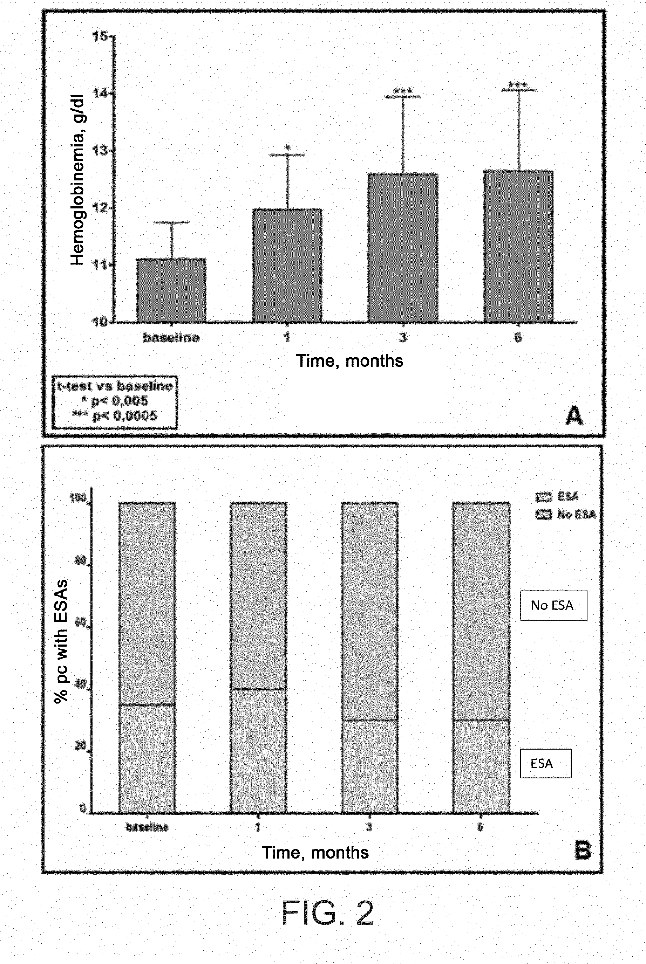

Results: At 4 weeks, serum iron showed significant changes (Fig. 1A), % transferrin saturation ($TSAT) (Fig. 1C) and hemoglobin (Fig. 2A) without significant changes in the % of patients on ESA therapy (Fig. 2B). Such changes remained significant at 6 months, especially regarding %TSAT. Ferritin showed no statistically significant changes (Fig. 1B).

Gastrointestinal tolerance was good. No patients experienced side effects or discontinued the therapy. There were no changes in renal and/or electrolyte function in the follow-up.

Conclusions: In stable KTx patients, treatment with liposomal divalent iron sulfate/vitamin C is effective and safe and represents a valid option for the correction of blood iron deficiency and consequent anemia in KTx patients.

### FORMULATION EXAMPLE

### Vegetable gelatin capsules containing:

| | |
|---|---|
| - liposomes containing iron sulfate 30 mg and vitamin C 24 mg | |
| - maltodextrins | 335 mg |
| - magnesium stearate | 10 mg |
| - silicon dioxide | 5 mg. |

## Claims

1. A liposomal composition comprising liposomes containing iron(II) sulfate and vitamin C, for use in the treatment of chronic renal failure.

2. The composition for use according to claim 1, wherein the treatment is administered to a population of patients who have undergone a kidney transplant.

3. The composition for use according to claim 1 or 2, wherein the liposomes form a liposomal component consisting of phospholipid vesicles, preferably lecithin phospholipids, containing per dose unit:
| | |
|---|---|
| - iron(II) sulfate | 20-40 mg; |
| - vitamin C | 20-30 mg. |

4. The composition for use according to claim 3, wherein, in addition to the aforesaid liposomal component, the composition comprises, per dose unit, 300-400 mg maltodextrins.

5. The composition for use according to any one of claims 1 to 4, wherein the composition, per dose unit, comprises or consists of:
- liposomes containing 30 mg iron(II) sulfate (150 mg 200 solution) and 24 mg vitamin C;
- 335 mg maltodextrins;
- non-active excipients.

6. The composition for use according to any one of claims 1 to 5, wherein said treatment causes an increase in serum iron, hemoglobin level, and transferrin saturation percentage, and does not cause gastrointestinal side effects.

7. A composition comprising or consisting of:
- liposomes containing iron(II) sulfate and vitamin C
- maltodextrins
- non-active excipients.

8. The composition according to claim 7, consisting of, per dose unit:
- liposomes containing 30 mg iron(II) sulfate (150 mg 200 solution) and 24 mg vitamin C;
- 335 mg maltodextrins;
- non-active excipients.

9. The composition according to claim 7 or 8, in the form of granules enclosed in gastro-resistant capsules.

10. The composition according to any one of claims 7 to 9, wherein said composition is formulated as a pharmaceutical product, a dietary product, food supplements, and foods for special medical purposes (FSMPs).
